# EUROPÄISCHE PATENTANMELDUNG

(11) **EP 2 305 262 A1**
(43) Veröffentlichungstag der Anmeldung: **06.04.2011**
(21) Anmeldenummer: 10184576.6
(22) Anmeldetag: 11.08.2003
(51) Int. Cl.: A61K 31/4985, A61K 31/00, A61P 25/00

(54) **Selektive Phosphodiesterase 9A-Inhibitoren als Arzneimittel zur Verbesserung kognitiver Prozesse**

(30) Priorität: 23.08.2002 DE 10238722
(62) Teilanmeldung aus: 03797233.8
(71) Anmelder: Boehringer Ingelheim International GmbH, 55216 Ingelheim am Rhein (DE)
(72) Erfinder: Böss, Frank-Gerhard, 60325, FRANKFURT (DE); Erb, Christina, 65189, WIESBADEN (DE); Hendrix, Martin, PINE BROOK, NJ 07058-9714 (US); Van Kampen, Marja, 41464, NEUSS (DE); Wunder, Frank, 42117, WUPPERTAL (DE)
(74) Vertreter: Hammann, Heinz

(57) **Zusammenfassung**

Die Erfindung betrifft die Verwendung von selektiven Phosphodiesterase 9A (PDE9A)-Inhibitoren zur Herstellung von Arzneimitteln zur Verbesserung von Wahrnehmung, Konzentrationsleistung, kognitiver Prozesse, Lernleistung und/oder Gedächtnisleistung.

SEQUENCE LISTING

<110>  Boehringer Ingelheim International GmbH, vormals Bayer AG, Bayer
Health Care

<120>  Selektive Phosphodiesterase 9A-Inhibitoren als Arzneimittel zur
Verbesserung kognitiver Prozesse

<130>  P01-2114/EP/2, vormals Le A 36 288

<160>  1

<170>  PatentIn version 3.1

<210>  1

<211>  1991

<212>  DNA

<213>  Homo sapiens

<400>  1
cgcggcggct ggcgtcggga aagtacagta aaaagtccga gtgcagccgc cgggcgcagg      60

atgggatccg gctcctccag ctaccggccc aaggccatct acctggacat cgatggacgc     120

attcagaagg taatcttcag caagtactgc aactccagcg acatcatgga cctgttctgc     180

atcgccaccg gcctgcctcg gaacacgacc atctccctgc tgaccaccga cgacgccatg     240

gtctccatcg accccaccat gcccgcgaat tcagaacgca ctccgtacaa agtgagacct     300

gtggccatca agcaactctc cgctggtgtc gaggacaaga gaaccacaag ccgtggccag     360

tctgctgaga gaccactgag ggacagacgg gttgtgggcc tggagcagcc ccggagggaa     420

ggagcatttg aaagtggaca ggtagagccc aggcccagag agccccaggg ctgctaccag     480

gaaggccagc gcatccctcc agagagagaa gaattaatcc agagcgtgct ggcgcaggtt     540

gcagagcagt tctcaagagc attcaaaatc aatgaactga aagctgaagt tgcaaatcac     600

ttggctgtcc tagagaaacg cgtggaattg gaaggactaa aagtggtgga gattgagaaa     660

tgcaagagtg acattaagaa gatgagggag gagctggcgg ccagaagcag caggaccaac     720

tgcccctgta agtacagttt tttggataac cacaagaagt tgactcctcg acgcgatgtt 780

cccacttacc ccaagtacct gctctctcca gagaccatcg aggccctgcg gaagccgacc 840

tttgacgtct ggctttggga gcccaatgag atgctgagct gcctggagca catgtaccac 900

gacctcgggc tggtcaggga cttcagcatc aaccctgtca ccctcaggag gtggctgttc 960

tgtgtccacg acaactacag aaacaacccc ttccacaact tccggcactg cttctgcgtg 1020

gcccagatga tgtacagcat ggtctggctc tgcagtctcc aggagaagtt ctcacaaacg 1080

gatatcctga tcctaatgac agcggccatc tgccacgatc tggaccatcc cggctacaac 1140

aacacgtacc agatcaatgc ccgcacagag ctggcggtcc gctacaatga catctcaccg 1200

ctggagaacc accactgcgc cgtggccttc cagatcctcg ccgagcctga gtgcaacatc 1260

ttctccaaca tcccacctga tgggttcaag cagatccgac agggaatgat cacattaatc 1320

ttggccactg acatggcaag acatgcagaa attatggatt ctttcaaaga gaaaatggag 1380

aattttgact acagcaacga ggagcacatg accctgctga agatgatttt gataaaatgc 1440

tgtgatatct ctaacgaggt ccgtccaatg gaagtcgcag agccttgggt ggactgttta 1500

ttagaggaat attttatgca gagcgaccgt gagaagtcag aaggccttcc tgtggcaccg 1560

ttcatggacc gagacaaagt gaccaaggcc acagcccaga ttgggttcat caagtttgtc 1620

ctgatcccaa tgtttgaaac agtgaccaag ctcttcccca tggttgagga gatcatgctg 1680

cagccacttt gggaatcccg agatcgctac gaggagctga agcggataga tgacgccatg 1740

aaagagttac agaagaagac tgacagcttg acgtctgggg ccaccgagaa gtccagagag 1800

agaagcagag atgtgaaaaa cagtgaagga gactgtgcct gaggaaagcg gggggcgtgg 1860

ctgcagttct ggacgggctg gccgagctgc gcgggatcct tgtgcaggga agagctgccc 1920

tgggcacctg gcaccacaag accatgtttt ctaagaacca ttttgttcac tgatacaaaa 1980

aaaaaaaaaa a 1991

## Beschreibung

Die Erfindung betrifft die Verwendung von selektiven Phosphodiesterase 9A (PDE 9A)-Inhibitoren zur Herstellung von Arzneimitteln zur Verbesserung von Wahrnehmung, Konzentrationsleistung, Lern- und/oder Gedächtnisleistung.

Die zelluläre Aktivierung von Adenylat- bzw. Guanylatzyklasen bewirkt die Zyklisierung von ATP bzw. GTP zu 5'-3' zyklischem Adenosin Monophosphat (cAMP) bzw. 5'-3' zyklischem Guanosin monophosphat (cGMP). Diese zyklischen Nukleotide (cAMP und cGMP) sind wichtige second messenger und spielen daher eine zentrale Rolle in den zellulären Signaltransduktionskaskaden. Beide aktivieren unter anderem, aber nicht ausschließlich, jeweils wieder Protein Kinasen. Die von cAMP aktivierte Protein Kinase wird Protein Kinase A (PKA) genannt, die von cGMP aktivierte Protein Kinase wird Protein Kinase G (PKG) genannt. Aktivierte PKA bzw. PKG können wiederum eine Reihe zellulärer Effektorproteine phosphorylieren (z.B. Ionenkanäle, G-Protein gekoppelte Rezeptoren, Strukturproteine). Auf diese Weise können die second messengers cAMP und cGMP die unterschiedlichsten physiologischen Vorgänge in den verschiedensten Organen kontrollieren. Die zyklischen Nukleotide können aber auch direkt auf Effektormoleküle wirken. So ist z.B. bekannt, dass cGMP direkt auf Ionenkanäle wirken kann und hiermit die zelluläre Ionenkonzentration beeinflussen kann (Übersicht in: Wei et al., Prog. Neurobiol., 1998, 56: 37 - 64). Ein Kontrollmechanismus, um die Aktivität von cAMP und cGMP und damit diese physiologischen Vorgänge wiederum zu steuern, sind die Phosphodiesterasen (PDE). PDEs hydrolysieren die zyklischen Monophosphate zu den inaktiven Monophosphaten AMP und GMP. Es sind mittlerweile mindestens 21 PDE Gene beschrieben (Exp. Opin. Investig. Drugs 2000, 9, 1354-3784). Diese 21 PDE Gene lassen sich aufgrund ihrer Sequenzhomologie in 11 PDE Familien einteilen (Nomenklatur Vorschlag siehe http://depts.washington.edu/pde/Nomenclature.html.). Einzelne PDE Gene innerhalb einer Familie werden durch Buchstaben unterschieden (z.B. PDE1A und PDE1B). Falls noch unterschiedliche Splice Varianten innerhalb eines Genes vorkommen, wird dies dann durch eine zusätzliche Nummerierung nach dem Buchstaben angegeben (z.B. PDE1A1).

### Abbildungen:

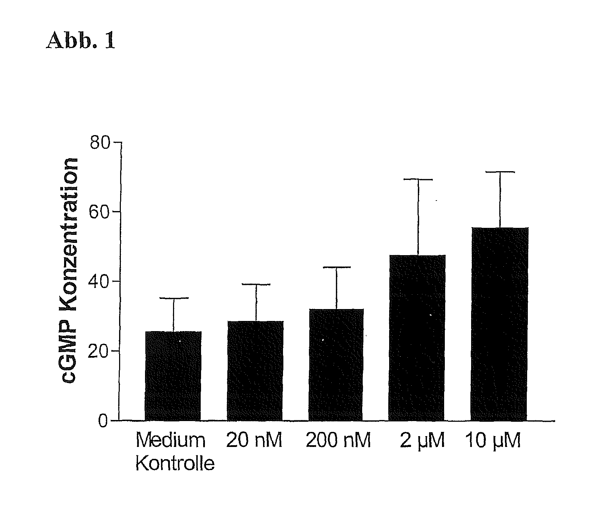

**Abb. 1****:** Intrazelluläre cGMP-Konzentration in primären Ratten (E18)-Kortex-kulturen nach Behandlung mit Beispiel 2. Die Zellen wurden 20 min mit Beispiel 2 in den angegebenen Konzentrationen inkubiert. Die Mediumkontrolle gibt den cGMP Wert in unbehandelten Zellen an. Der DMSO Gehalt betrug in den Mediumkontrollen wie in den substanzbehandelten Zellen 0.2 %. Die cGMP Konzentration ist aufgetragen in fmol/150000 Zellen.

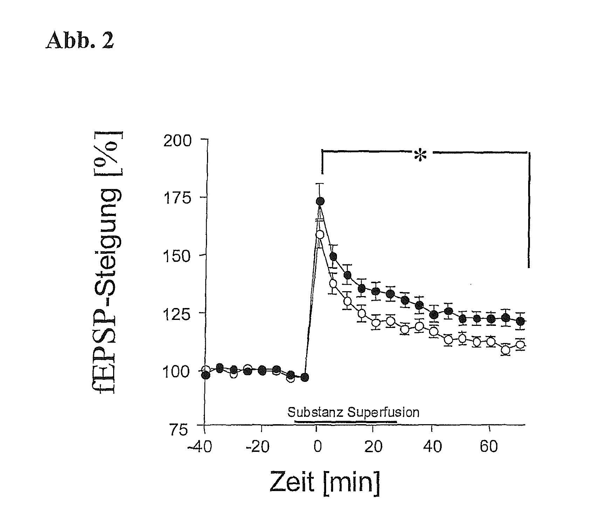

**Abb. 2****:** Effekt von Beispiel 2 (10 µM) auf die Potenzierung des fokalen exzitatorischen postsynaptischen Potentials (fEPSP) nach schwachem Tetanus. Beispiel 2 verstärkte den Anstieg des fEPSPs signifikant, wenn es 10 Minuten vor bis 30 Minuten nach einem schwachen Tetanus eingewaschen wurde (geschlossene Symbole). Die Kontrolle wurde mit 0.01 % DMSO behandelt (offene Symbole). *: p < 0.05; Varianzanalyse mit den Faktoren Dosierung (0 und 10 µM) und Testzeitpunkt mit dem Faktor Messzeitpunkt.

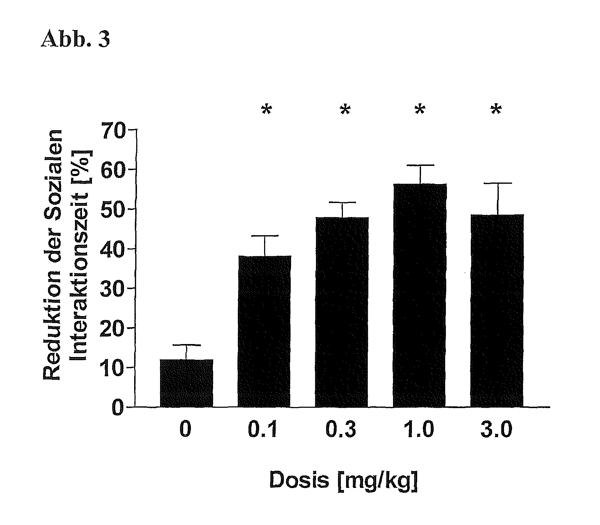

**Abb. 3****:** Wirkung von Beispiel 2 auf die prozentuale Reduktion (Trial 2/Trial 1) der Interaktionszeit im Sozialen Wiedererkennungstest (Mittelwert + S.E.M). Den Tieren wurde Vehikel (10 % Ethanol, 20 % Solutol, 70 % physiologische Kochsalzlösung) oder 0,1 mg/kg, 0,3 mg/kg, 1,0 mg/kg bzw. 3,0 mg/kg von Beispiel 2 intraperitoneal direkt nach dem ersten Zusammentreffen (Trial 1) injiziert. Statistische Auswertung: **p* < 0.05.

### PDE9A - Entdeckung, Eigenschaften, Verteilung

Die Humane PDE9A (GenBank/EMBL Accession Number NM_002606, cDNA Sequenz siehe Sequence Listing, SEQ ID NO:1) wurde 1998 kloniert und sequenziert. Die Aminosäurenidentität zu anderen PDEs liegt bei maximal 34 % (PDE8A) und minimal 28% (PDE5A). Mit einem Km-Wert von 170 nM ist PDE9A hochaffin für cGMP. Darüber hinaus ist PDE9A selektiv für cGMP (Km-Wert für cAMP = 230 µM). PDE9A weist keine cGMP Bindungsdomäne auf (GAF-Domäne), die auf eine allosterische Enzymregulation durch cGMP schließen ließe. In einer Western Blot Analyse wurde gezeigt, dass die PDE9A im Mensch in Hoden, Gehirn, Dünndarm, Skelettmuskulatur, Herz, Lunge, Thymus und Milz exprimiert wird. Die höchste Expression wurde in Gehirn, Dünndarm, Herz und Milz gefunden (Fisher et al., J. Biol. Chem, 1998, 273 (25): 15559 - 15564). Das Gen für die humane PDE9A liegt auf Chromosom 21q22.3 und enthält 20 Exons. Bislang wurden 20 alternative Spleißvarianten der PDE9A identifiziert (Guipponi et al., Hum. Genet., 1998, 103: 386 - 392; Rentero et al., Biochem. Biophys. Res. Commun., 2003, 301: 686 - 692). Klassische PDE Inhibitoren hemmen die humane PDE9A nicht. So zeigen IBMX, Dipyridamole, SKF94120, Rolipram und Vinpocetin in Konzentrationen bis 100 µM keine Inhibition am isolierten Enzym. Für Zaprinast wurde ein IC₅₀-Wert von 35 µM nachgewiesen (Fisher et al., J. Biol. Chem, 1998, 273 (25): 15559 -15564).

Die Maus PDE9A wurde 1998 von Soderling et al. (J. Biol. Chem, 1998, 273 (19): 15553 - 15558) kloniert und sequenziert. Diese ist wie die humane Form hochaffin für cGMP mit einem Km-Wert von 70 nM. In der Maus wurde eine besonders hohe Expression in Niere, Gehirn, Lunge und Herz gefunden. Auch die Maus PDE9A wird von IBMX in Konzentrationen unter 200 µM nicht gehemmt; der IC₅₀ für Zaprinast liegt bei 29 µM (Soderling et al., J. Biol. Chem, 1998, 273 (19): 15553 -15558). Im Rattengehirn wurde gezeigt, dass PDE9A in einigen Hirnregionen stark exprimiert wird. Dazu zählen der Bulbus olfactorius, Hippocampus, Cortex, Basalganglien und basales Vorderhirn (Andreeva et al., J. Neurosci., 2001, 21 (22): 9068 - 9076). Insbesondere Hippokampus, Cortex und basales Vorderhirn spielen eine wichtige Rolle in Lern- und Gedächtnisvorgängen.

PDE9A zeichnet sich durch eine besonders hohe Affinität für cGMP aus. Der Km-Wert für cGMP beträgt 170 nM (Fisher et al., J. Biol. Chem., 1998, 273 (25): 15559 - 15564). Deshalb ist PDE9A im Gegensatz zu PDE2A (Km = 10 µM; Martins et al., J. Biol. Chem., 1982, 257: 1973 - 1979), PDE5A (Km = 4 µM; Francis et al., J. Biol. Chem., 1980, 255: 620 - 626), PDE6A (Km = 17 µM; Gillespie and Beavo, J. Biol. Chem., 1988, 263 (17): 8133 - 8141) und PDE11A (Km = 0,52 µM; Fawcett et al., PNAS, 2000, 97 (7): 3702 - 3707) schon bei niedrigen physiologischen Konzentrationen aktiv. Im Gegensatz zu PDE2A (Murashima et al., Biochemistry, 1990, 29: 5285 - 5292) wird die katalytische Aktvität von PDE9A nicht durch cGMP gesteigert, da es keine GAF Domäne aufweist (Beavo et al., Current Opinion in Cell Biology, 2000, 12: 174 -179). PDE9A Inhibitoren führen deshalb zu einer Erhöhung der basalen cGMP Konzentration (siehe Abbildung 1). Diese Erhöhung der basalen cGMP Konzentration führte überraschenderweise zu einer Verbesserung der Lern- und Gedächtnisleistung im Social Recognition Test.

Überraschenderweise wurde nachgewiesen, dass PDE9A Inhibitoren eine Wirkung auf die Funktion des zentralen Nervensystems haben. Insbesondere wurde nun gefunden, dass selektive PDE9A-Inhibitoren zur Herstellung von Arzneimitteln zur Verbesserung der Wahrnehmung, Konzentrationsleistung, Lernleistung oder Gedächtnisleistung geeignet sind.

Ein PDE9A-Inhibitor im Sinne der Erfindung ist eine Verbindung, die humane PDE 9A unter den unten angegebenen Bedingungen mit einem IC₅₀ von weniger als 10 µM, bevorzugt weniger als 1µM hemmt.

Ein selektiver PDE9A-Inhibitor im Sinne der Erfindung ist eine Verbindung, die humane PDE9A unter den unten angegebenen Bedingungen stärker hemmt als die humanen PDE1C, PDE2A, PDE3B, PDE4B, PDE5A, PDE7B, PDE8A,PDE10A und PDE11A. Bevorzugt ist IC₅₀ (PDE9A)/ IC₅₀ (PDE1C, PDE2A, PDE3B, PDE4B, PDE5A,PDE7B, PDE8A,PDE10A und PDE11A) kleiner als 0,2.

Besonders eignen sich die selektiven PDE9A-Inhibitoren zur Verbesserung der Wahrnehmung, Konzentrationsleistung, Lernleistung, oder Gedächtnisleistung nach Kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatische Demenz, allgemeine Konzentrationsstörungen, Konzentrationsstörungen in Kindern mit Lern- und Gedächtnisproblemen, Alzheimersche Krankheit, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyotrope Lateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die Erfindung betrifft die Verwendung von selektiven PDE9A-Inhibitoren zur Herstellung von Arzneimitteln zur Verbesserung der Wahrnehmung, Konzentrationsleistung, kognitiver Prozesse, Lernleistung und/oder Gedächtnisleistung.

Des weiteren betrifft die Erfindung die Verwendung von selektiven PDE9A-Inhibitoren zur Prophylaxe und/oder Behandlung von Störungen der Wahrnehmung, Konzentrationsleistung, kognitiver Prozesse, Lernleistung und/oder Gedächtnisleistung.

Dabei kann die Störung unter anderem eine Folge einer Erkrankung ausgewählt aus der Gruppe Demenz, Schlaganfall, Schädel-Hirn-Trauma, Alzheimersche Krankheit, Parkinsonsche Krankheit, Depression, oder Demenz mit Frontallappendegeneration sein.

Des weiteren betrifft die Erfindung die Verwendung von PDE9A Inhibitoren zur Behandlung von Krankheiten des Zentralen Nervensystems, die durch Beeinflußung der cGMP-Spiegel therapiert werden können. So betrifft die Erfindung zum Beispiel die Behandlung von Demenz, Schlaganfall Schädel-Hirn-Trauma, Alzheimersche Krankheit, Demenz mit Frontallappendegeneration, Lewy-Body-Demenz, vaskuläre Demenz, Attention-Deficit-Syndrome, Aufmerksamkeits- und Konzentrationsstörungen, Parkinsonsche Krankheit, Schizophrenie, Depression, affektive Erkrankungen, Psychosen, Neurosen, Angst, Manie oder manisch-depressive Erkrankungen, Morbus Pick, Schmerz und Epilepsie.

Die Erfindung betrifft bevorzugt die erfindungsgemäße Verwendung der PDE9A-Inhibitoren der Formeln

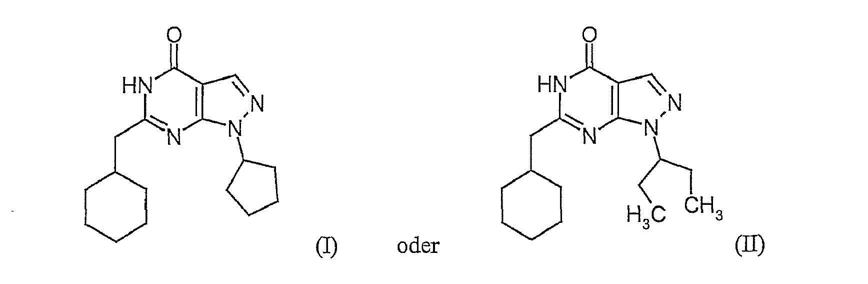

Die Verbindungen der Formeln (I) und (II) können auch in Form ihrer Salze, Solvate oder Solvate ihrer Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren, wie beispielsweise Chlorwasserstoffsäure, Bromwasserstoffsäure, Phosphorsäure oder Schwefelsäure, oder Salze mit organischen Carbonoder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind Alkalimetallsalze (z.B. Natrium- oder Kaliumsalze), Erdalkalisalze (z.B. Magnesium- oder Calciumsalze), sowie Ammoniumsalze, die abgeleitet sind von Ammoniak oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die WO 98/40384 offenbart Pyrazolopyrimidine, die sich als PDE1-, 2- und 5-Inhibitoren auszeichnen und für die Behandlung von cardiovasculären, cerebrovasculären Erkrankungen sowie Erkrankungen des Urogenitalbereiches eingesetzt werden können.

In CH 396 924, CH 396 925, CH 396 926, CH 396 927, DE 1 147 234, DE 1 149 013, GB 937,726 werden Pyrazolopyrimidine mit coronarerweiternder Wirkung beschrieben, die zur Behandlung von Durchblutungsstörungen des Herzmuskels eingesetzt werden können.

Im US 3,732,225 werden Pyrazolopyrimidine beschrieben, die eine entzündungshemmende und Blutzucker-senkende Wirkung haben.

In DE 2 408 906 werden Styrolpyrazolpyrimidine beschrieben, die als antimikrobielle und entzündungshemmende Mittel für die Behandlung von beispielsweise Ödem eingesetzt werden können.

Die erfindungsgemäßen Verbindungen können über das im Folgenden beschriebene Syntheseschema hergestellt werden:

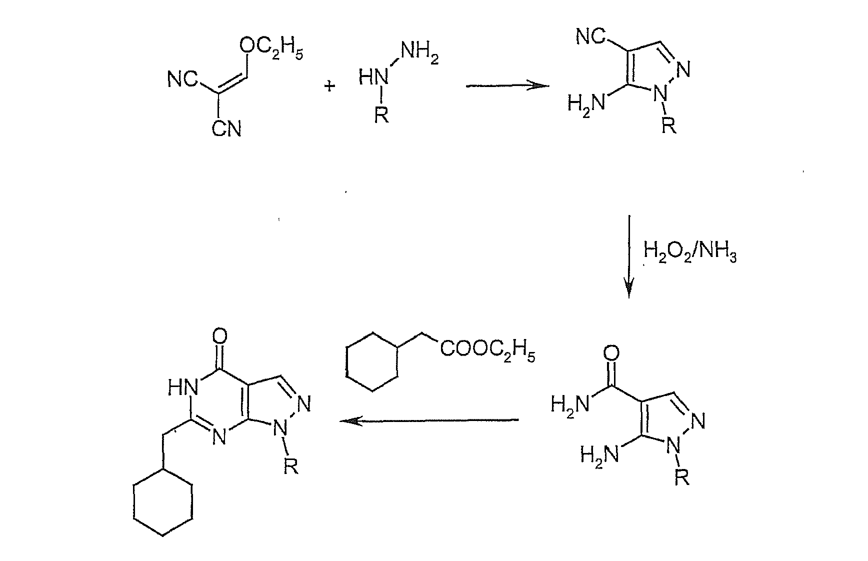

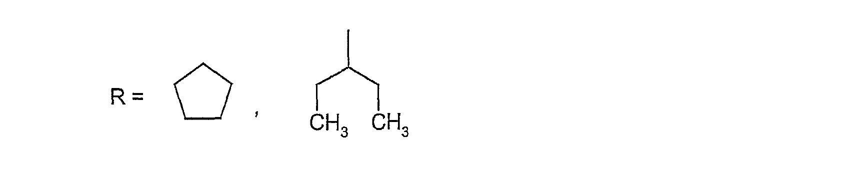

Eine gegebenenfalls anschließende Umsetzung der Verbindungen der Formel (I) und (II) mit den entsprechenden (i) Lösungsmitteln und/oder (ii) Basen oder Säuren führt zu den entsprechenden Salzen, Solvaten und/oder Solvaten der Salze.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rektal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. magensaftresistente Überzüge), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im Allgemeinen hat es sich als vorteilhaft erwiesen, bei parenteraler Applikation Mengen von etwa 0,001 bis 30 mg/kg, vorzugsweise etwa 0,01 bis 10 mg/kg Körpergewicht zur Erzielung wirksamer Ergebnisse zu verabreichen. Bei oraler Applikation beträgt die Menge etwa 0,01 bis 100 mg/kg, vorzugsweise etwa 0,1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

### PDE-Inhibition

Rekombinante PDE1C (GenBank/EMBL Accession Number: NM_005020, Loughney et al. J. Biol. Chem. 1996 271, 796-806), PDE2A (GenBank/EMBL Accession Number: NM_002599, Rosman et al. Gene 1997 191, 89-95), PDE3B (GenBank/EMBL Accession Number: NM_000922, Miki et al. Genomics 1996 36, 476-485), PDE4B (GenBank/EMBL Accession Number: NM_002600, Obernolte et al. Gene. 1993 129, 239-247), PDE5A (GenBank/EMBL Accession Number: NM_001083, Loughney et al. Gene 1998 216, 139-147), PDE7B (GenBank/EMBL Accession Number: NM_018945, Hetman et al. Proc. Natl. Acad. Sci. U.S.A. 2000 97, 472-476), PDE8A (GenBank/EMBL Accession Number: AF_056490, Fisher et al. Biochem. Biophys. Res. Commun. 1998 246, 570-577), PDE9A (GenBank/EMBL Accession Number NM_002606, cDNA Sequenz siehe Sequence Listing, SEQ ID NO:1, Fisher et al., J. Biol. Chem., 1998, 273 (25): 15559 - 15564), PDE10A (GenBank/EMBL Accession Number: NM_06661, Fujishige et al. J. Biol. Chem. 1999 274, 18438-45.), PDE11A (GenBank/EMBL Accession Number: NM_016953, Fawcett et al. Proc. Natl. Acad. Sci. 2000, 97, 3702-3707) wurden mit Hilfe des pFASTBAC Baculovirus Expressionssystems (GibcoBRL) in Sf9 Zellen exprimiert. 48 h nach der Infektion werden die Zellen geerntet und in 20 mL (pro 1L Kultur) Lysispuffer (50 mM Tris-HCl, pH 7.4, 50 mM NaCl, 1 mM MgCl₂, 1.5 mM EDTA, 10% Glycerin plus 20 µL Protease Inhibitor Cocktail Set III [CalBiochem, La Jolla, CA USA]) suspendiert. Die Zellen werden bei 4°C für 1 Minute mit Ultraschall behandelt und anschließend für 30 Minuten bei 4°C mit 10000 Upm zentrifugiert. Der Überstand (PDE Präparat) wurde gesammelt und bei -20°C aufbewahrt.

Die Testsubstanzen werden zur Bestimmung ihrer *in vitro* Wirkung an PDE 9A in 100% DMSO aufgelöst und seriell verdünnt. Typischerweise werden Verdünnungsreihen von 200 µM bis 1.6 µM hergestellt (resultierende Endkonzentrationen im Test: 4 µM bis 0.032 µM). Jeweils 2 µL der verdünnten Substanzlösungen werden in die Vertiefungen von Mikrotiterplatten (Isoplate; Wallac Inc., Atlanta, GA) vorgelegt. Anschließend werden 50 µL einer Verdünnung des oben beschriebenen PDE9A Präparates hinzugefügt. Die Verdünnung des PDE9A Präparates wird so gewählt, dass während der späteren Inkubation weniger als 70% des Substrates umgesetzt wird (typische Verdünnung: 1: 10000; Verdünnungspuffer: 50 mM Tris/HCl pH 7.5, 8.3 mM MgCl₂, 1.7 mM EDTA, 0.2% BSA). Das Substrat, [8-³H] guanosine 3', 5'-cyclic phosphate (1 µCi/µL; Amersham Pharmacia Biotech., Piscataway, NJ) wird 1:2000 mit Assaypuffer (50 mM Tris/HCl pH 7.5, 8.3 mM MgCl₂, 1.7 mM EDTA) auf eine Konzentration von 0.0005µCi/µL verdünnt. Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µl eines in Assaypuffer gelösten PDE9A-Inhibitors (z.B. der Inhibitor aus Herstellbeispiel 2, 10 µM Endkonzentration) gestoppt. Direkt im Anschluss werden 25 µL einer Suspension mit 18 mg/mL Yttrium Scintillation Proximity Beads (Amersham Pharmacia Biotech., Piscataway, NJ.) hinzugefügt. Die Mikrotiterplatten werden mit einer Folie versiegelt und für 60 min bei Raumtemperatur stehen gelassen. Anschließend werden die Platten für 30 s pro Vertiefung in einem Microbeta Szintillationzähler (Wallac Inc., Atlanta, GA) vermessen. IC₅₀-Werte werden anhand der graphischen Auftragung der Substanzkonzentration gegen die prozentuale Inhibition bestimmt.

### Die in vitro Wirkung von Testsubstanzen an rekombinanter PDE3B, PDE4B, PDE7B, PDE8A, PDE10A und PDE11A wird nach dem oben für PDE 9A beschriebenen Testprotokoll mit folgenden Anpassungen bestimmt: Als Substrat wird [5',8-³H] adenosine 3', 5'-cyclic phosphate (1 µCi/µL; Amersham Pharmacia Biotech., Piscataway, NJ) verwendet. Die Zugabe einer Inhibitorlösung zum Stoppen der Reaktion ist nicht notwendig. Stattdessen wird in Anschluss an die Inkubation von Substrat und PDE direkt mit der Zugabe der Yttrium Scintillation Proximity Beads wie oben beschrieben fortgefahren und dadurch die Reaktion gestoppt. Für die Bestimmung einer entsprechenden Wirkung an rekombinanter PDE1C, PDE2A und PDE5A wird das Protokoll zusätzlich wie folgt angepasst: Bei PDE1C werden zusätzlich Calmodulin 10⁻⁷ M und CaCl₂ 3mM zum Reaktionsansatz gegeben. PDE2A wird im Test durch Zugabe von cGMP 1 µM stimuliert und mit einer BSA Konzentration von 0,01 % getestet. Für PDE1C und PDE2A wird als Substrat [5',8-³H] adenosine 3', 5'-cyclic phosphate (1 µCi/µL; Amersham Pharmacia Biotech., Piscataway, NJ), Für PDE5A [8-³H] guanosine 3', 5'-cyclic phosphate (1 µCi/µL; Amersham Pharmacia Biotech., Piscataway, NJ) eingesetzt.

### Inhibition von PDE-Isoenzymen durch Beispiel 2:

| Isoenzym | Species | IC₅₀ [nM] |
|---|---|---|
| PDE1C | human | 720 |
| PDE2A | human | > 4000 |
| PDE3B | human | > 4000 |
| PDE4B | human | > 4000 |
| PDE5A | human | > 4000 |
| PDE7B | human | > 4000 |
| PDE8A | human | > 4000 |
| PDE9A | human | 110 |
| PDE10A | human | > 4000 |
| PDB11 | human | > 4000 |

Die PDE9A-inhibierende Wirkung von Beispiel 1 kann durch einen IC₅₀-Wert von IC₅₀ = 5 nM experimentell belegt werden.

### Erhöhung der intrazellulären neuronalen cGMP-Konzentration in Zellkulturen

PDE9A-Inhibitoren erhöhen die intrazelluläre neuronale cGMP in kultivierten primären kortikalen Neuronen.

Rattenembryonen (Embryonaltag E17 - E19) wurden dekapitiert, die Köpfe in mit Präparationsmedium (DMEM, Penicillin/Streptomycin; beides von Gibco) gefüllte Präparationsschalen überführt. Die Kopfhaut und Schädeldecke wurde entfernt, und die freipräparierten Gehirne wurden in eine weitere Petrischale mit Präparationsmedium überführt. Mithilfe eines Binokulars und zweier Pinzetten wurde das Großhirn (Kortex) isoliert und mit Eis auf 4°C gekühlt. Diese Präparation und die Vereinzelung der kortikalen Neuronen wurden dann nach einem Standardprotokoll mit dem Papain-Kit (Worthington Biochemical Corporation, Lakewood, New Jersey 08701, USA) durchgeführt (Huettner et al. J. Neurosci. 1986, 6, 3044-3060.). Die mechanisch vereinzelten kortikalen Neurone wurden zu 150.000 Zellen/Loch in 200 µl Neurobasalmedium/Loch (Neurobasal; B27 Supplement; 2 mM L-Glutamin; in Anwesenheit von Penicillin/Streptomycin; alle Agenzien von Gibco) 7 Tage in 96 Lochplatten (mit Poly-D Lysin 100 µg/ml für 30 min vorbehandelt) unter Standard Bedingungen kultiviert (37°C, 5 % CO₂). Nach 7 Tagen wurde das Medium abgenommen und die Zellen mit HBSS Puffer (Hank's balanced salt solution, Gibco/BRL) gewaschen. Anschließend wurden 100 µl Testsubstanz Beispiel 2 in HBSS Puffer gelöst (zuvor in 100 % DMSO gelöst: 10 mM) auf die Zellen gegeben. Anschließend wurden nochmals 100 µl HBSS Puffer zugegeben, sodass die Endkonzentration der Testsubstanz Beispiel 2 so lag wie in Abb. 1 angegeben und bei 37°C für 20 min inkubiert. Der Testpuffer wurde danach komplett abgenommen. Anschließend wurden die Zellen in 200 µl Lysispuffer (cGMP Kit code RPN 226; von Amersham Pharmacia Biotech.) lysiert und die cGMP Konzentration nach den Angaben des Herstellers gemessen. Alle Messungen wurden in Triplikaten durchgeführt. Die statistische Auswertung erfolgte mit Prism Software Version 2.0 (GraphPad Software Inc., San Diego, CA USA).

Inkubation der primären Neuronen mit Beispiel 2 führte zu einer Steigerung des cGMP Gehaltes (Abb. 1).

### Langzeitpotenzierung

Langzeitpotenzierung wird als ein zelluläres Korrelat für Lern- und Gedächtnisvorgänge angesehen. Zur Bestimmung, ob PDE9 Inhibition einen Einfluss auf Langzeitpotenzierung hat, wurde folgende Methode angewandt:

Rattenhippokampi wurden in einen Winkel von etwa 70 Grad im Verhältnis zur Schnittklinge plaziert (Chopper). In Abständen von 400 µm wurde der Hippokampus geschnitten. Die Schnitte wurden mit Hilfe eines sehr weichen, stark benetzten Pinsels (Marderhaar) von der Klinge genommen und in ein Glasgefäß mit carbogenisierter gekühlter Nährlösung (124 mM NaCl, 4,9 mM KCl, 1,3 mM MgSO₄* 7H₂O, 2,5 mM CaCl²⁺ Wasser- frei, 1,2 mM KH₂PO₄, 25,6 mM NaHCO₃, 10 mM Glucose, pH 7.4) überführt. Während der Messung befanden sich die Schnitte in einer temperierten Kammer unter einem Flüssigkeitsspiegel von 1-3 mm Höhe. Die Durchflussrate betrug 2,5 ml/min. Die Vorbegasung erfolgte unter geringen Überdruck (etwa 1 atm) sowie über eine Mikrokanüle in der Vorkammer. Die Schnittkammer war mit der Vorkammer so verbunden, dass eine Minizirkulation aufrechterhalten werden konnte. Als Antrieb der Minizirkulation wurde das durch die Mikrokanüle ausströmende Carbogen eingesetzt. Die frisch präparierten Hippokampusschnitte wurden mindestens 1 Stunde bei 33°C in der Schnittkammer adaptiert.

Die Reizstärke wurde so gewählt, dass die fokalen exzitatiorischen postsynaptischen Potentiale (FFPSP) 30 % des maximalen exzitatorischen postsynaptischen Potentials (EPSP) betrugen. Mit Hilfe einer monopolaren Stimulationselektrode, die aus lackiertem Edelstahl bestand und eines stromkonstanten, biphasischen Reizgenerators (AM-Systems 2100), wurden lokal die Schaffer-Kollateralen erregt (Spannung: 1-5 V, Impulsbreite einer Polarität 0.1 ms, Gesamtimpuls 0.2 ms). Mit Hilfe von Glaselektroden (Borosilikatglas mit Filament, 1-5 MOhm, Durchmesser: 1.5 mm, Spitzendurchmesser: 3-20 µm), die mit normaler Nährlösung gefüllt waren, wurden aus dem Stratum radiatum die exzitatorischen postsynaptischen Potentiale (fEPSP) registriert. Die Messung der Feldpotentiale geschah gegenüber einer chlorierten Referenzelektrode aus Silber, die sich am Rande der Schnittkammer befand, mit Hilfe eines Gleichspannungsverstärkers. Das Filtern der Feldpotentiale erfolgte über einen Low-Pass Filter (5 kHz). Für die statistische Analyse der Experimente wurde der Anstieg (slope) der fEPSPs (fEPSP-Anstieg) ermittelt. Die Aufnahme, Analyse und Steuerung des Experimentes erfolgte mit Hilfe eines Softwareprogrammes (PWIN), welches in der Abteilung Neurophysiologie des Leibniz-Institutes für Neurobiologie, Magdeburg entwickelt wurde. Die Mittelwertbildung der fEPSP-Anstiegswerte zu den jeweiligen Zeitpunkten und die Konstruktion der Diagramme erfolgte mit Hilfe der Software EXCEL, wobei ein entsprechendes Makro die Aufnahme der Daten automatisierte.

In den Kontrollexperimenten wurde zunächst für 60 - 120 Minuten die basale synaptisch Transmission registriert. -Anschließend wurden im Abstand von 200 ms viermal zwei Doppelpulse mit einem Interpulsabstand der Doppelpulse von 10 ms und einer Breite der Einzelpulse von 0,2 ms (schwacher Tetanus) appliziert (Zeitpunkt = 0 Minuten in Abbildung 2). Die resultierende Potenzierung der EPSPs wurde für mindestens 60 Minuten aufgezeichnet. Beispiel 2 wurde im dargestellten Experiment 10 Minuten vor bis 30 Minuten nach der Stimulation eingespült.

Superfusion der Hippokampusschnitte mit einer 10 µM Lösung von Beispiel 2 führte zu einer signifikanten Steigerung der LTP (Abbildung 2).

### Sozialer Wiedererkennungstest:

Der Soziale Wiedererkennungstest ist ein Lern- und Gedächtnistest. Er misst die Fähigkeit von Ratten, zwischen bekannten und unbekannten Artgenossen zu unterscheiden. Deshalb eignet sich dieser Test zur Prüfung der lern- oder gedächtnisverbessernden Wirkung von Testsubstanzen.

Erwachsene Ratten, die in Gruppen gehalten wurden, wurden 30 Minuten vor Testbeginn einzeln in Testkäfige gesetzt. Vier Minuten vor Testbeginn wurde das

Testtier in eine Beobachtungsbox gebracht. Nach dieser Adaptationszeit wurde ein juveniles Tier zu dem Testtier gesetzt und 2 Minuten lang die absolute Zeit gemessen, die das adulte Tier das Junge inspiziert (Trial 1). Gemessen wurden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hatte. Danach wurde das Juvenile herausgenommen, das Adulte mit Beispiel 2 oder Vehikel behandelt und anschließend in seinen Heimkäfig zurückgesetzt. Nach einer Retentionszeit von 24 Stunden wurde der Test wiederholt (Trial 2). Eine verringerte Soziale Interaktionszeit im Vergleich zu Trial 1 würde anzeigen, dass die adulte Ratte sich an das Jungtier erinnert.

Die adulten Tiere wurden direkt im Anschluss an Trial 1 entweder mit Vehikel (10% Ethanol, 20 % Solutol, 70 % physiologische Kochsalzlösung) oder 0,1 mg/kg, 0,3 mg/kg, 1,0 mg/kg bzw. 3,0 mg/kg von Beispiel 2 gelöst in 10 % Ethanol, 20 % Solutol, 70 % physiologische Kochsalzlösung intraperitoneal injiziert. Vehikel behandelte Ratten zeigten keine Reduktion der sozialen Interaktionszeit in Trial 2 verglichen mit Trial 1. Sie hatten folglich vergessen, dass sie schon einmal Kontakt mit dem Jungtier hatten. Überraschenderweise war die soziale Interaktionszeit im zweiten Durchgang nach Behandlung Beispiel 2 signifikant gegenüber den Vehikel behandelten reduziert. Dies bedeutet, dass die substanzbehandelten Ratten sich an das juvenile Tier erinnert haben und somit Beispiel 2 eine verbessernde Wirkung auf Lernen und Gedächtnis aufwies.

Verwendete Abkürzungen:
- DMSO: Dimethylsulfoxid
- d.Th.: der Theorie (bei Ausbeute)
- equiv.: Äquivalent(e)
- ESI: Elektrospray-Ionisation (bei MS)
- HPLC: Hochdruck-, Hochleistungsflüssigchromatographie
- MS: Massenspektroskopie
- NMR: Kernresonanzspektroskopie
- Smp.: Schmelzpunkt

### Ausführungsbeispiele:

### Beispiel 1

6-(Cyclohexylmethyl)-1-cyclopentyl-1,5-dihydro-4H-pyrazolo[3,4-d]pyrimidin-4-on

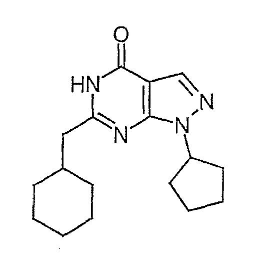

### Stufe 1a)

5-Amino-1-cyclopentyl-1H-pyrazol-4-carbonitril

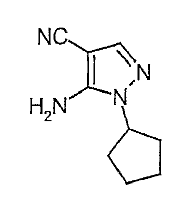

Eine Lösung von Ethoxymethylenmalonsäuredinitril (7.93 g, 64.9 mmol) in 100 ml Methanol wird bei Raumtemperatur unter Argon langsam mit Cyclopentylhydrazin (6.5 g, 64.9 mmol) versetzt, anschließend 3 h unter Rückfluss erhitzt und dann über Nacht bei Raumtemperatur gerührt. Das Lösungsmittel wird am Rotationsverdampfer abgezogen und der Rückstand mit Diethylether verrührt. Der Feststoff wird abgesaugt, mit Diethylether gewaschen und im Hochvakuum getrocknet.

Ausbeute: 7.24 g (63% d.Th.)

MS (ESI): m/z = 177 (M+H)⁺

¹H-NMR (200 MHz, CDCl₃): δ = 7.5 (s, 1H), 4.45 (br.s, 2H), 4.35 (m, 1H), 2.2-1.55 (m, 6H) ppm.

### Stufe 1b)

5-Amino-1-cyclopentyl-1H-pyrazol-4-carboxamid

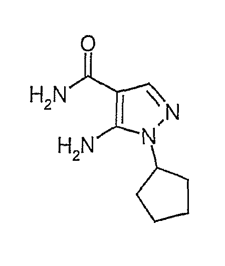

Eine Lösung von 5-Amino-1-cyclopentyl-1H-pyrazol-4-carbonitril (6.74 g, 38.3 mmol) in einem Gemisch aus 300 ml Ethanol und 371 ml konzentrierter wässriger Ammoniaklösung wird bei Raumtemperatur mit 85 ml 30%-iger Wasserstoffperoxidlösung versetzt und über Nacht bei Raumtemperatur gerührt. Anschließend werden am Rotationsverdampfer die nichtwässrigen Lösemittel abgezogen. Aus der verbleibenden Mischung fällt das Produkt als Feststoff aus, der abgesaugt, mit Diethylether gewaschen und im Hochvakuum getrocknet wird.

Ausbeute: 5.31 g (71% d.Th.)

MS (ESI): m/z = 195 (M+H)⁺

¹H-NMR (200 MHz, CDCl₃): δ = 7.5 (s, 1H), 5.6-4.8 (breit, 4H), 4.35 (m, 1H), 2.2-1.55 (m, 8H) ppm.

### Stufe 1c)

6-(Cyclohexylmethyl)-1-cyclopentyl-1,5-dihydro-4H-pyrazolo[3,4-d]pyrimidin-4-on

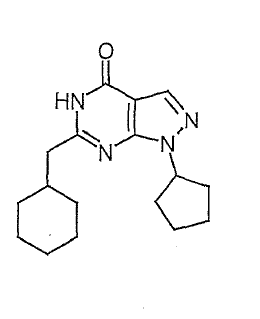

Unter Argon werden 75 mg (0.39 mmol) 5-Amino-1-cyclopentyl-1H-pyrazol-4-carboxamid und 183 mg (1.16 mmol, 3 equiv.) Cyclohexylessigsäuremethylester in 1.5 ml absolutem Ethanol vorgelegt. Bei 0°C werden 54 mg Natriumhydrid (60%-ige Dispersion in Mineralöl; 1.35 mmol, 3.5 equiv.) im Argon-Gegenstrom langsam zugegeben. Das entstandene Gemisch wird langsam erwärmt und für 18 h unter Rückfluss gerührt. Zur Aufarbeitung werden 20 ml Wasser zugegeben und das Gemisch mehrmals mit Essigsäureethylester extrahiert. Die vereinigten organischen Phasen werden über Natriumsulfat getrocknet und im Vakuum eingeengt. Das Rohprodukt wird mittels präparativer HPLC gereinigt.

Ausbeute: 36 mg (31% d.Th.)

MS (ESI): m/z = 301 (M+H)⁺

Smp.: 147°C

¹H-NMR (300 MHz, DMSO-d₆): δ = 11.95 (s, 1H), 8.0 (s, 1H), 5.1 (m, 1H), 2.5 (d, 2H), 2.15-1.75 (m, 7H), 1.75-1.55 (m, 7H), 1.3-0.9 (m, 5H) ppm.

### Beispiel 2

**6-(Cyclohexylmethyl)-1-(1-ethylpropyl)-1,5-dihydro-4H-pyrazolo[3,4-d]-pyrimidin-4-on**

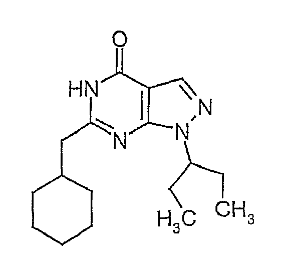

### Stufe 2α)

5-Amino-1-(1-ethylpropyl)-1H-pyrazol-4-carbonitril

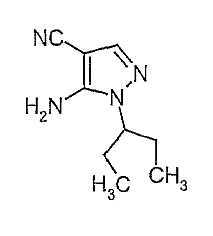

Die Herstellung erfolgt analog der Vorschrift für Beispiel 1 / Stufe 1a).

MS (ESI): m/z = 179 (M+H)⁺

¹H-NMR (300 MHz, DMSO-d₆): δ = 7.55 (s, 1H), 6.45 (s, 2H), 4.0 (m, 1H), 1.8-1.55 (m, 4H), 0.65 (t, 6H) ppm.

### Stufe 2b)

5-Amino-1-(1-ethylpropyl)-1H-pyrazol-4-carboxamid

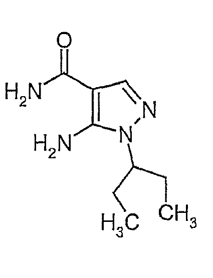

Die Herstellung erfolgt analog der Vorschrift für Beispiel 1 / Stufe 1b)

MS (ESI): m/z = 197 (M+H)⁺

¹H-NMR (300 MHz, DMSO-d₆): δ = 7.65 (s, 1H), 6.9 (br. s, 2H), 6.1 (s, 2H), 3.9 (m, 1H), 1.85-1.6 (m, 4H), 0.7 (t, 6H) ppm.

### Stufe 2c)

6-(Cyclohexylmethyl)-1-(1-ethylpropyl)-1,5-dihydro-4H-pyrazolo[3,4-d]pyrimidin-4-on

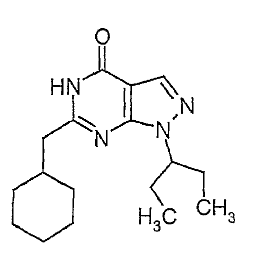

Analog zu Beispiel 1 / Stufe 1c) wird das Produkt ausgehend von 200 mg (1.02 mmol) 5-Amino-1-(1-ethylpropyl)-1H-pyrazol-4-carboxamid und 482 mg (3.06 mmol) Cyclohexylessigsäuremethylester erhalten.

Ausbeute: 146 mg (47% d.Th.)

MS (ESI): m/z = 303 (M+H)⁺

Smp.: 122°C

¹H-NMR (200 MHz, DMSO-d₆): δ = 12.0 (s, 1H), 8.0 (s, 1H), 4.45 (m, 1H), 2.5 (m, 2H), 2.0-1.5 (m, 10H), 1.4-0.9 (m, 5H), 0.6 (t, 6H, *J=* 7.5 Hz) ppm.

## Patentansprüche

1. Verwendung von selektiven PDE9A-Inhibitoren zur Herstellung von Arzneimitteln zur Verbesserung der Wahrnehmung, Konzentrationsleistung, kognitiver Prozesse, Lernleistung und/oder Gedächtnisleistung.

2. Verwendung nach Anspruch 1 zur Prophylaxe und/oder Behandlung von Störungen der Wahrnehmung, Konzentrationsleistung, kognitiver Prozesse, Lernleistung und/oder Gedächtnisleistung.

3. Verwendung nach Anspruch 2, wobei die Störung eine Folge einer Erkrankung ausgewählt aus einer Gruppe bestehend aus Demenz, Schlaganfall, Schädel-Hirn-Trauma, Alzheimersche Krankheit, Parkinsonsche Krankheit, Depression, oder Demenz mit Frontallappendegeneration ist.

4. Verwendung von PDE9A-Inhibitoren zur Herstellung von Arzneimitteln zur Behandlung und/oder Prophylaxe von Krankheiten des Zentralen Nervensystems, die durch Beeinflussung der cGMP-Spiegel therapiert werden können.

5. Verwendung nach Anspruch 4, wobei die Krankheit aus einer Gruppe von Krankheiten bestehend aus Demenz, Schlaganfall Schädel-Hirn-Trauma, Alzheimersche Krankheit, Demenz mit Frontallappendegeneration, Lewy-Body-Demenz, vaskuläre Demenz, Attention-Deficit-Syndrome, Aufmerksamkeits- und Konzentrationsstörungen, Parkinsonsche Krankheit, Schizophrenie, Depression, affektive Erkrankungen, Psychosen, Neurosen, Angst, Manie oder manisch-depressive Erkrankungen, Morbus Pick, Schmerz und Epilepsie ausgewählt wird.

6. Verwendung nach einem der Ansprüche 1 bis 5, wobei der selektive PDE9A-Inhibitor eine Verbindung der Formel 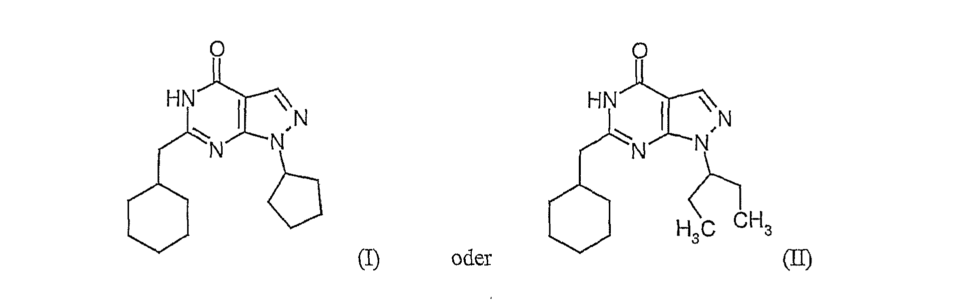 sowie deren Salze, Solvate und Solvate der Salze ist.
